# EUROPEAN PATENT APPLICATION

(11) **EP 4 809 830 A1**
(43) Date of publication of application: **23.09.2026**
(21) Application number: 25760953.7
(22) Date of filing: 26.02.2025
(51) Int. Cl.: A23L 33/125, A61K 31/702, A61P 1/00, A23C 9/13, A23C 9/156, A23C 9/16, A23L 2/00

(54) **USE OF 2'-FUCOSYLLACTOSE IN PROMOTING INTESTINAL DEVELOPMENT AND PREVENTING INTESTINAL DAMAGE**

(30) Priority: 28.02.2024 CN 202410220178
(71) Applicant: Ausnutria Dairy (China) Co., Ltd., Changsha, Hunan 410127 (CN)
(72) Inventor: WA, Yunchao, Changsha, Hunan 410127 (CN); PENG, Xiaoyu, Changsha, Hunan 410127 (CN); LIU, Min, Changsha, Hunan 410127 (CN); ZHAO, Xia, Changsha, Hunan 410127 (CN); XIE, Yu, Changsha, Hunan 410127 (CN); GU, Ruixia, Changsha, Hunan 410127 (CN); YANG, Zhenquan, Changsha, Hunan 410127 (CN); PAN, Lina, Changsha, Hunan 410127 (CN); WANG, Jiaqi, Changsha, Hunan 410127 (CN); LI, Wei, Changsha, Hunan 410127 (CN)
(74) Representative: Comoglio, Elena
(86) International application number: PCT/CN2025/079329
(87) International publication number: WO 2025/180413

(57) **Abstract**

The use of 2'-fucosyllactose in the preparation of a food/drug for promoting intestinal glycocalyx development and preventing intestinal glycocalyx damage caused by lipopolysaccharide (LPS). The use of 2'-fucosyllactose in the preparation of a food/drug for the adjuvant treatment and/or adjuvant prevention of gastrointestinal infections, inflammatory bowel diseases, obesity or allergy caused by glycocalyx damage. Before adherent growth of Caco-2 cells, adding 2'-FL for co-culture with the Caco-2 cells can simulate the intestinal growth and maturation state. In the state, 2'-FL can significantly promote the development of the glycocalyx layer of Caco-2 cells, and can further prevent the glycocalyx layer damage caused by LPS on this basis.

## Description

### CROSS-REFERENCE TO THE RELATED APPLICATIONS

The present invention claims priority to Chinese Patent Application No. 202410220178.0, filed with the China National Intellectual Property Administration on February 28, 2024, and entitled "USE OF 2'-FUCOSYLLACTOSE IN PROMOTING INTESTINAL DEVELOPMENT AND PREVENTING INTESTINAL DAMAGE", which is incorporated herein by reference in entirety.

### TECHNICAL FIELD

The present invention relates to the technical field of human milk oligosaccharides, and specifically relates to use of 2'-fucosyllactose in promoting intestinal development and preventing intestinal damage.

### BACKGROUND

An intestinal barrier is considered a gatekeeper of human health. The intestinal barrier is responsible for the passage and absorption of nutrients, but it also prevents pathogens from entering the human body. Furthermore, it regulates interference between the intestinal lumen microbiota and macromolecules, and maintains tolerance and immune responses. After birth, a gatekeeping function of the intestinal barrier has not yet fully developed. Any disruption to a neonatal intestinal barrier function can have long-term effects and may play a role in the development of gastrointestinal infections and inflammatory bowel diseases (IBD). In addition, it can affect the development of other diseases in childhood and adulthood, such as obesity and allergies.

Human milk is a gold standard of infant nutrition, and human milk oligosaccharides (HMOs) thereof are a main component that distinguish human milk from milk of other mammals. In infant formula milk powder, an important function of the HMOs is to stimulate colonization of microbiota in a gastrointestinal tract and promote the development of intestinal barrier function.

Glycocalyx on the neonatal intestinal epithelium provides binding sites for symbiotic microorganisms. Well-developed glycocalyx can prevent pathogen adhesion and act as a barrier against luminal toxins and enzymes. Glycocalyx is composed of glycans and proteins. Proteoglycans are generally considered as the most important component of glycocalyx and form framework of glycocalyx. In addition, glycocalyx contains glycosaminoglycan chains linked to a core protein of the proteoglycans. Heparan sulfate (HS) and hyaluronic acid (HA) are main glycosaminoglycan components in glycocalyx.

Currently, there are few clinical intervention studies on direct regulatory effects of HMOs on intestinal cells and structures. Further research is necessary to lay a theoretical foundation for future applications of HMOs.

### SUMMARY

In view of this, the present invention provides use of 2'-fucosyllactose in promoting intestinal glycocalyx development and preventing intestinal glycocalyx damage. The present invention investigates effects of 2'-fucosyllactose (2'-FL) on development and function of glycocalyx in intestinal epithelial cells Caco-2 at a cellular level.

To address the technical problems raised in the background art, the present invention adopts following technical solution.

In the first aspect, the present invention provides use of 2'-fucosyllactose in preparation of a food for promoting intestinal glycocalyx development and/or preventing glycocalyx damage caused by lipopolysaccharide (LPS).

In an embodiment, the food includes at least one of infant formula milk powder, milk powder for kids, adult milk powder, a food for a special medical purpose, a nutritional product, liquid milk, a complementary food, and a non-alcoholic beverage.

In the second aspect, the present invention provides use of 2'-fucosyllactose in preparation of a drug for promoting intestinal glycocalyx development and/or preventing glycocalyx damage caused by lipopolysaccharide (LPS).

In the third aspect, the present invention provides use of 2'-fucosyllactose in promoting intestinal glycocalyx development and/or preventing glycocalyx damage caused by lipopolysaccharide (LPS).

In an embodiment, the drug further includes other acceptable carriers and/or excipients.

In an embodiment, a performance of promoting intestinal glycocalyx development is at least one selected from:
protecting a protein backbone of glycocalyx, maintaining normal synthesis of hyaluronic acid of glycocalyx, and maintaining normal synthesis of heparan sulfate of glycocalyx.

In an embodiment, the performance of promoting intestinal glycocalyx development is at least one selected from: upregulating a transcriptional level of glycocalyx development-related gene glypican 1, upregulating a transcriptional level of a gene encoding hyaluronic acid synthase, and upregulating a transcriptional level of a gene encoding exostosin glycosyltransferase.

In an embodiment, the performance of promoting intestinal glycocalyx development is at least one selected from: upregulating a level of *gpc1* mRNA, a level of *has1* mRNA, a level of *has2* mRNA, a level of *has3* mRNA, a level of *ext1* mRNA, and a level of *ext2* mRNA.

In an embodiment, a performance of preventing glycocalyx damage caused by lipopolysaccharide (LPS) is at least one selected from:
preventing damage of protein backbone of glycocalyx caused by LPS, preventing damage of hyaluronic acid synthase of glycocalyx caused by LPS, and preventing damage of heparan sulfate of glycocalyx caused by LPS.

In an embodiment, the performance of preventing glycocalyx damage caused by lipopolysaccharide (LPS) is at least one selected from:
upregulating a decline in the transcriptional level of glycocalyx development-related gene glypican 1 caused by LPS, upregulating a decline in the transcriptional level of the gene encoding hyaluronic acid synthase caused by LPS, and upregulating a decline in the transcriptional level of the gene encoding exostosin glycosyltransferase caused by LPS.

In an embodiment, the performance of preventing glycocalyx damage caused by lipopolysaccharide (LPS) is selected from upregulating a decline in a level of at least one of following mRNAs caused by LPS:
*gpc1* mRNA, *has1* mRNA, *has2* mRNA, *has3* mRNA, *ext1* mRNA, and *ext2* mRNA.

In an embodiment, in use of 2'-fucosyllactose in preparation of a food for promoting intestinal glycocalyx development, a use concentration of 2'-fucosyllactose is 0.6 mg/mL-2.4 mg/mL, optionally, the use concentration of 2'-fucosyllactose is 0.6 mg/mL-1.2 mg/mL, and optionally, the use concentration of 2'-fucosyllactose is 1.2 mg/mL-2.4 mg/mL.

In an embodiment, the food includes at least one of infant formula milk powder, milk powder for kids, adult milk powder, a food for a special medical purpose, a nutritional product, liquid milk, a complementary food, and a non-alcoholic beverage.

In an embodiment, in use of 2'-fucosyllactose in preparation of a drug for promoting intestinal glycocalyx development, a use concentration of 2'-fucosyllactose is 0.6 mg/mL-2.4 mg/mL, and optionally, the use concentration of 2'-fucosyllactose is 0.6 mg/mL-1.2 mg/mL.

In an embodiment, in use of 2'-fucosyllactose in the preparation of a food for preventing glycocalyx damage caused by lipopolysaccharide (LPS), a use concentration of 2'-fucosyllactose is 0.6 mg/mL-2.4 mg/mL, and optionally, the use concentration of 2'-fucosyllactose is 0.6 mg/mL-1.2 mg/mL.

In an embodiment, in use of 2'-fucosyllactose in preparation of a drug for preventing glycocalyx damage caused by lipopolysaccharide (LPS), a use concentration of 2'-fucosyllactose is 0.6 mg/mL-2.4 mg/mL, and optionally, the use concentration of 2'-fucosyllactose is 0.6 mg/mL-1.2 mg/mL.

In the fourth aspect, the present invention provides use of 2'-fucosyllactose in preparation of a food for an adjunctive treatment and/or adjunctive prevention of a gastrointestinal infection, an inflammatory bowel disease, obesity, or allergy caused by glycocalyx damage.

In the fifth aspect, the present invention provides use of 2'-fucosyllactose in preparation of a drug for the treatment and/or prevention of a gastrointestinal infection, an inflammatory bowel disease, obesity, or allergy caused by glycocalyx damage.

In the sixth aspect, the present invention provides use of 2'-fucosyllactose in the adjunctive treatment and/or adjunctive prevention of a gastrointestinal infection, an inflammatory bowel disease, obesity, or allergy caused by glycocalyx damage.

In an embodiment, in use of 2'-fucosyllactose in preparation of a food for the adjunctive treatment and/or adjunctive prevention of a gastrointestinal infection, an inflammatory bowel disease, obesity, or allergy caused by glycocalyx damage, a use concentration of 2'-fucosyllactose is 0.6 mg/mL-2.4 mg/mL, and optionally, the use concentration of 2'-fucosyllactose is 0.6 mg/mL-1.2 mg/mL.

In an embodiment, in use of 2'-fucosyllactose in preparation of a health food for the adjunctive treatment and/or adjunctive prevention of a gastrointestinal infection, an inflammatory bowel disease, obesity, or allergy caused by glycocalyx damage, a use concentration of 2'-fucosyllactose is 0.6 mg/mL-2.4 mg/mL, and optionally, the use concentration of 2'-fucosyllactose is 0.6 mg/mL-1.2 mg/mL.

In an embodiment, in use of 2'-fucosyllactose in preparation of a drug for the treatment and/or prevention of a gastrointestinal infection, an inflammatory bowel disease, obesity, or allergy caused by glycocalyx damage, a use concentration of 2'-fucosyllactose is 0.6 mg/mL-2.4 mg/mL, and optionally, the use concentration of 2'-fucosyllactose is 0.6 mg/mL-1.2 mg/mL.

The beneficial effects of the above technical solution of the present invention are as follows.

The present invention provides use of 2'-fucosyllactose. Before adherent growth of Caco-2 cells herein, adding 2'-FL for co-culture with the Caco-2 cells can simulate the state of the intestine when growth has matured. In the state, 2'-FL can significantly promote the development of the glycocalyx layer of Caco-2 cells, and can further prevent the glycocalyx layer damage caused by LPS on this basis, showing that supplementing with a specific dose of 2'-FL during an intestinal maturation stage can significantly promote development of the intestinal glycocalyx layer and promote overall healthy development of the intestine, preventing damage from external factors such as LPS.

### BRIEF DESCRIPTION OF DRAWINGS

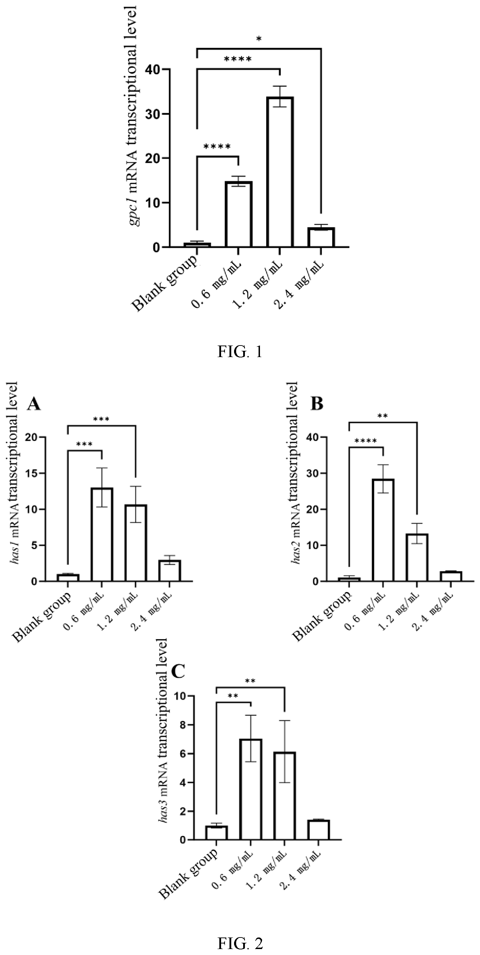

FIG. 1 shows effects of different concentrations of 2'-FL on a transcriptional level of *gpc1* mRNA;
FIG. 2 shows effects of different concentrations of 2'-FL on transcriptional levels of *has1* mRNA, *has2* mRNA, and *has3* mRNA, wherein A represents effects of different concentrations of 2'-FL on the transcriptional level of *has1* mRNA, B represents effects of different concentrations of 2'-FL on the transcriptional level of *has2* mRNA, and C represents effects of different concentrations of 2'-FL on the transcriptional level of *has3* mRNA;

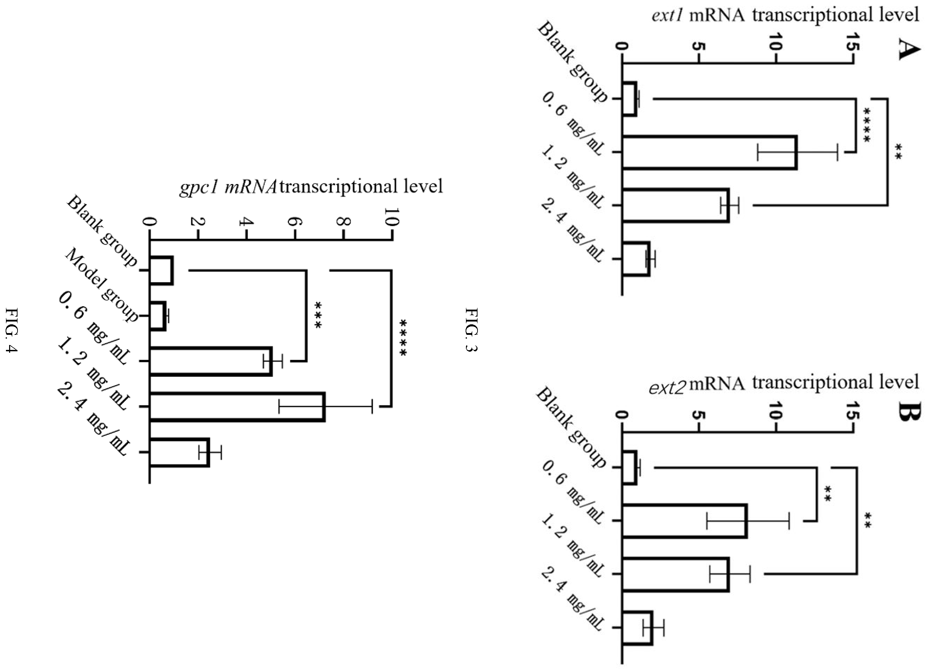

FIG. 3 shows effects of different concentrations of 2'-FL on transcriptional levels of *ext1* mRNA and *ext2* mRNA, wherein A represents the effects of different concentrations of 2'-FL on the transcriptional level of *ext1* mRNA, and B represents the effects of different concentrations of 2'-FL on the transcriptional level of *ext2* mRNA;
FIG. 4 shows effects of different concentrations of 2'-FL and LPS on a transcriptional level of *gpc1* mRNA;

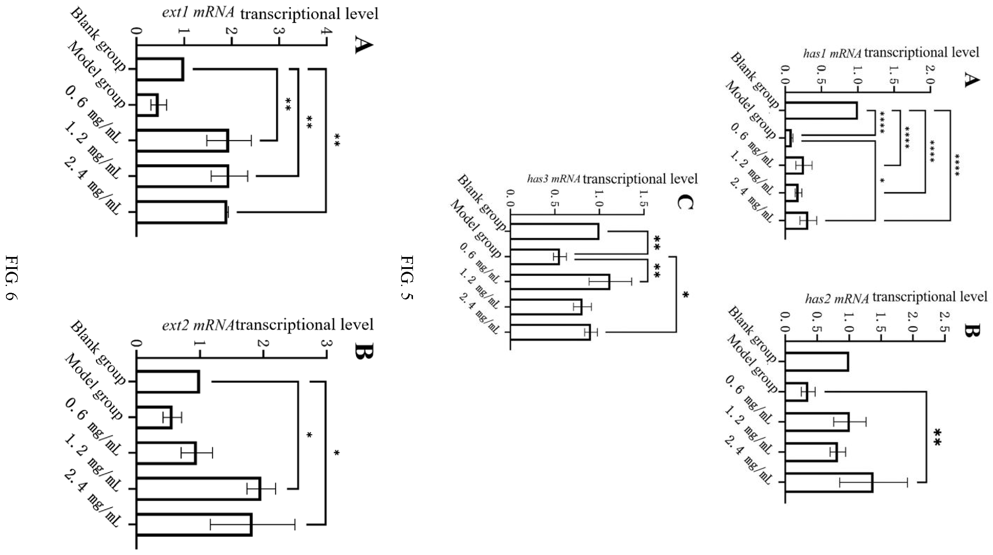

FIG. 5 shows effects of different concentrations of 2'-FL and LPS on the transcriptional levels of *has1* mRNA, *has2* mRNA, and *has3* mRNA, wherein A represents the effects of different concentrations of 2'-FL and LPS on the transcriptional level of *has1* mRNA, B represents the effects of different concentrations of 2'-FL and LPS on the transcriptional level of *has2* mRNA, and C represents the effects of different concentrations of 2'-FL and LPS on the transcriptional level of *has3* mRNA; and
FIG. 6 shows effects of different concentrations of 2'-FL and LPS on transcriptional levels of *ext1* mRNA and *ext2* mRNA, wherein A represents the effects of different concentrations of 2'-FL and LPS on the transcriptional level of *ext1* mRNA, and B represents the effects of different concentrations of 2'-FL and LPS on the transcriptional level of *ext2* mRNA.

### DETAILED DESCRIPTION OF EMBODIMENTS

To further understand the present invention, preferred embodiments herein are described below in conjunction with examples. However, it should be understood that these descriptions are only for further illustrating features and advantages of the present invention and are not intended to limit the present invention.

In the first aspect, the present invention provides use of 2'-fucosyllactose in preparation of a food for promoting intestinal glycocalyx development and/or preventing glycocalyx damage caused by lipopolysaccharide (LPS).

According to some embodiments herein, the food includes at least one of infant formula milk powder, milk powder for kids, adult milk powder, a food for a special medical purpose, a nutritional product, liquid milk, a complementary food, and a non-alcoholic beverage.

In the second aspect, the present invention provides use of 2'-fucosyllactose in the preparation of a drug for promoting intestinal glycocalyx development and/or preventing glycocalyx damage caused by lipopolysaccharide (LPS).

According to some embodiment herein, the drug further includes other acceptable carriers and/or excipients.

In the third aspect, the present invention provides use of 2'-fucosyllactose in promoting intestinal glycocalyx development and/or preventing glycocalyx damage caused by lipopolysaccharide (LPS).

In an embodiment, a performance of promoting intestinal glycocalyx development is at least one selected from:
protecting a protein backbone of glycocalyx, maintaining normal synthesis of hyaluronic acid of glycocalyx, and maintaining normal synthesis of heparan sulfate of glycocalyx.

In an embodiment, the performance of promoting intestinal glycocalyx development is at least one selected from: upregulating a transcriptional level of glycocalyx development-related gene glypican 1, upregulating a transcriptional level of a gene encoding hyaluronic acid synthase, and upregulating a transcriptional level of a gene encoding exostosin glycosyltransferase.

In an embodiment, the performance of promoting intestinal glycocalyx development is at least one selected from: upregulating a level of *gpc1* mRNA, a level of *has1* mRNA, a level of *has2* mRNA, a level of *has3* mRNA, a level of *ext1* mRNA, and a level of *ext2* mRNA.

In an embodiment, the performance of preventing glycocalyx damage caused by lipopolysaccharide (LPS) is at least one selected from:
preventing damage of protein backbone of glycocalyx caused by LPS, preventing damage of hyaluronic acid synthase of glycocalyx caused by LPS, and preventing damage of heparan sulfate of glycocalyx caused by LPS. In an embodiment, the performance of preventing glycocalyx damage caused by lipopolysaccharide (LPS) includes, but is not limited to, restoring and maintaining the glycocalyx.

In an embodiment, the performance of the preventing glycocalyx damage caused by lipopolysaccharide (LPS) is at least one selected from:
upregulating a decline in the transcriptional level of glycocalyx development-related gene glypican 1 caused by LPS, upregulating a decline in the transcriptional level of the gene encoding hyaluronic acid synthase caused by LPS, and upregulating a decline in the transcriptional level of the gene encoding exostosin glycosyltransferase caused by LPS.

In an embodiment, the performance of the preventing glycocalyx damage caused by lipopolysaccharide (LPS) is selected from upregulating a decline in a level of at least one of following mRNAs caused by LPS:
*gpc1* mRNA, *has1* mRNA, *has2* mRNA, *has3* mRNA, *ext1* mRNA, and *ext2* mRNA.

According to some embodiments of the present invention, in use of 2'-fucosyllactose in preparation of a food for promoting intestinal glycocalyx development, the use concentration of 2'-fucosyllactose is 0.6 mg/mL-2.4 mg/mL, optionally, the use concentration of 2'-fucosyllactose is 0.6 mg/mL-1.2 mg/mL. Optionally, the use concentration of 2'-fucosyllactose is 1.2 mg/mL-2.4 mg/mL. 2'-fucosyllactose at 0.6 mg/mL can significantly upregulate the transcriptional levels of *has1, has2,* and *has3*; 2'-fucosyllactose at 0.6 mg/mL-1.2 mg/mL can significantly upregulate the transcriptional level of *gpc1*; and 2'-fucosyllactose at 0.6 mg/mL can significantly upregulate the transcriptional levels of *ext1* and *ext2*. 2'-fucosyllactose at 0.6mg/mL-1.2 mg/mL can significantly prevent downregulation of the transcriptional level of *gpc1* caused by LPS, thereby protecting the protein backbone of glycocalyx; 2'-fucosyllactose at 0.6mg/mL-2.4 mg/mL can significantly prevent downregulation of the transcriptional levels of *has2* and *has3* caused by LPS, thereby maintaining synthesis of hyaluronic acid of glycocalyx; and 2'-Fucosyllactose at 0.6mg/mL-2.4 mg/mL can significantly prevent downregulation of the transcriptional levels of *ext1* and *ext2* caused by LPS, thereby maintaining the synthesis of heparan sulfate of glycocalyx.

According to some embodiments of the present invention, in use of 2'-fucosyllactose in preparation of a health food for promoting intestinal glycocalyx development, the use concentration of 2'-fucosyllactose is 0.6 mg/mL-2.4 mg/mL, and optionally, the use concentration of 2'-fucosyllactose is 0.6 mg/mL-1.2 mg/mL.

According to some embodiments of the present invention, in use of 2'-fucosyllactose in preparation of a drug for promoting intestinal glycocalyx development, the use concentration of 2'-fucosyllactose is 0.6 mg/mL-2.4 mg/mL, and optionally, the use concentration of 2'-fucosyllactose is 0.6 mg/mL-1.2 mg/mL.

According to some embodiments of the present invention, in use of 2'-fucosyllactose in preparation of a food for preventing glycocalyx damage caused by lipopolysaccharide (LPS), a use concentration of 2'-fucosyllactose is 0.6 mg/mL-2.4 mg/mL, and optionally, the use concentration of 2'-fucosyllactose is 0.6 mg/mL-1.2 mg/mL.

According to some embodiments of the present invention, in use of 2'-fucosyllactose in the preparation of a health food for preventing glycocalyx damage caused by lipopolysaccharide (LPS), a use concentration of 2'-fucosyllactose is 0.6 mg/mL-2.4 mg/mL, and optionally, the use concentration of 2'-fucosyllactose is 0.6 mg/mL-1.2 mg/mL.

According to some embodiments of the present invention, in use of 2'-fucosyllactose in preparation of a drug for preventing glycocalyx damage caused by lipopolysaccharide (LPS), a use concentration of 2'-fucosyllactose is 0.6 mg/mL-2.4 mg/mL, and optionally, the use concentration of 2'-fucosyllactose is 0.6 mg/mL-1.2 mg/mL.

In the fourth aspect, the present invention provides use of 2'-fucosyllactose in preparation of a food for the adjunctive treatment and/or adjunctive prevention of a gastrointestinal infection, an inflammatory bowel disease, obesity, and allergy caused by glycocalyx damage.

According to some embodiments of the present invention, the food includes at least one of infant formula milk powder, milk powder for kids, adult milk powder, a food for a special medical purpose, a nutritional product, liquid milk, a complementary food, and a non-alcoholic beverage.

In the fifth aspect, the present invention provides use of 2'-fucosyllactose in preparation of a drug for the treatment and/or prevention of a gastrointestinal infection, an inflammatory bowel disease, obesity, or allergy caused by glycocalyx damage.

In the sixth aspect, the present invention provides use of 2'-fucosyllactose in the adjunctive treatment and/or adjunctive prevention of a gastrointestinal infection, an inflammatory bowel disease, obesity, or allergy caused by glycocalyx damage.

The inflammatory bowel disease is any one selected from the group consisting of acute colitis, ulcerative colitis, Crohn's disease, microscopic colitis, diversion colitis, Behcet's disease immuno-oncology colitis, chemotherapy or radiation colitis, graft-versus-host disease colitis, collagenous colitis, lymphocytic colitis, and pouchitis.

According to some embodiments of the present invention, in use of 2'-fucosyllactose in preparation of a food for the adjunctive treatment and/or adjunctive prevention of a gastrointestinal infection, an inflammatory bowel disease, obesity, or allergy caused by glycocalyx damage, a use concentration of 2'-fucosyllactose is 0.6 mg/mL-2.4 mg/mL, and optionally, the use concentration of 2'-fucosyllactose is 0.6 mg/mL-1.2 mg/mL.

According to some embodiments of the present invention, in use of 2'-fucosyllactose in preparation of a health food for the adjunctive treatment and/or adjunctive prevention of a gastrointestinal infection, an inflammatory bowel disease, obesity, or allergy caused by glycocalyx damage, a use concentration of 2'-fucosyllactose is 0.6 mg/mL-2.4 mg/mL, and optionally, the use concentration of 2'-fucosyllactose is 0.6 mg/mL-1.2 mg/mL.

According to some embodiments of the present invention, in use of 2'-fucosyllactose in preparation of a drug for the treatment and/or prevention of a gastrointestinal infection, an inflammatory bowel disease, obesity, or allergy caused by glycocalyx damage, a use concentration of 2'-fucosyllactose is 0.6 mg/mL-2.4 mg/mL, and optionally, the use concentration of 2'-fucosyllactose is 0.6 mg/mL-1.2 mg/mL.

The present invention is further illustrated below with some specific examples.

Raw materials used in following examples are as follows.

Human colon adenocarcinoma cells Caco-2: purchased from Nanjing SenBeiJia Biological Technology Co., Ltd.

MEM complete medium: purchased from Nanjing SenBeiJia Biological Technology Co., Ltd.

Modified MRS medium: purchased from Hope Bio-Technology Co., Ltd.

Lipopolysaccharide (LPS): purchased from Sigma-Aldrich (Shanghai) Trading Co., Ltd.

Caco-2 cell culture: human colon adenocarcinoma cells Caco-2 were cultured in MEM complete medium containing 20% high-quality fetal bovine serum, grown in 25 cm² cell culture flasks and incubated in a carbon dioxide incubator at 37 °C, 5% CO₂, and 95% relative humidity. The culture medium was changed every one or two days. When the cells reach 80%-90% confluence, the cells were digested with 0.25% trypsin digestion solution and subcultured at a ratio of 1:3.

### Example 1

### Effects of 2 '-FL on glycocalyx development in intestinal epithelial cells

1.1 Experiments: Caco-2 cells were adjusted to a density of 1 × 10⁵ cells/cm² and inoculated in 24-well plates. According to groups, 2'-FL at concentrations of 0.6 mg/mL, 1.2 mg/mL, and 2.4 mg/mL was added to MEM complete medium, respectively. After incubation for 3 days, MEM medium was served as a blank control. Total RNA was extracted from the cells and reverse transcribed. Transcriptional levels of genes related to glycocalyx development, including glypican 1 (*gpc1*), hyaluronic acid synthases (*has1*, *has2* and *has3*), and exostosin glycosyltransferases (*ext1* and *ext2*) in the Caco-2 cells were measured by qPCR. qPCR primer sequences were shown in Table 1.

qPCR kit manufacturer: Nanjing Vazyme Biotechnology Co., Ltd.; Catalog number: AceQ Universal U+ Probe Master Mix V2; amplification program: step 1: 37 °C for 2 min, and cycle number 1; step 2: 95 °C for 5 min, and cycle number 1; and step 3: 95 °C for 10 s, 60°C for 30 s, and cycle number 45.

**Table 1**

| Name | Sequence Number | Upstream | Sequence Number | Downstream |
|---|---|---|---|---|
| *gpc1* | SEQ:1 | 5’- TATTGCCGAAATGT GCTCAAGGGC | SEQ:7 | 5’- ATGACACTCTCCAC ACCCGATGTA |
| *has1* | SEQ:2 | 5’- CCACCCAGTACAGC GTCAAC | SEQ:8 | 5'- CATGGTGCTTCTGT CGCTCT |
| *has2* | SEQ:3 | 5’- TCTTTATGTGACTC ATCTGTCTCACCGG | SEQ:9 | 5’- TTGTTGGCTACCAG TTTATCCAAACG |
| *has3* | SEQ:4 | 5’- | SEQ:10 | 5'- |
| | | TATACCGCGCTC CAA | | GCCACTCCCGGAAG TAAGACT |
| *ext1* | SEQ:5 | 5’- CATAGGCGATGAG AGATTGT | SEQ:11 | 5’- CAAGAATTGTGTCT GCTGTC |
| *ext2* | SEQ:6 | 5'- GGCTACGATGTCAG CATTCCTG | SEQ:12 | 5’- GGCTTCTAGGTCCT CTCTGTAC |

### 1.2 Test Results

### 1.2.1 Effects of different concentrations of 2'-FL on protein backbone of glycocalyx

*gpc1* is a gene encoding proteoglycan Glypican 1 (GPC1). GPC1 is the protein backbone of glycocalyx and an important carrier of glycosaminoglycan chains, including HA and heparan sulfate (HS). HA is a highly viscous component of an intestinal mucus layer, responsible for tissue repair, stability, and anti-inflammatory functions. FIG. 1 shows effects of different concentrations of 2'-FL on the transcriptional level of *gpc1* mRNA. As can be seen from FIG. 1, all three selected intervention concentrations of 2'-FL can significantly upregulate the transcriptional level of *gpc1* (*p* < 0.05). With increase in 2'-FL concentration, an upregulation effect on the transcriptional level of *gpc1* shows a trend of first increasing and then decreasing, reaching a maximum at 1.2 mg/mL.

### 1.2.2 Effects of different concentrations of 2'-FL on hyaluronic acid synthases of glycocalyx

*has1*, *has2*, and *has3* are genes encoding hyaluronic acid (HA) synthase. HAS is crucial for synthesis of HA, and HA is a highly viscous component of the intestinal mucus layer, responsible for tissue repair, stability, and anti-inflammatory effects. Effects of HAS1 and HAS2 primarily catalyze a polymerization reaction between glucosamine and UDP-glucose, thereby synthesizing hyaluronic acid. Hyaluronic acid plays important structural and biological functions in the extracellular matrix, including intercellular signaling, cell migration, tissue repair, etc. Compared to HAS1 and HAS2, HAS3 synthesizes low molecular weight HA, and the low molecular weight HA is essential for the development of intestinal stem cells. FIG. 2 shows effects of different concentrations of 2'-FL on transcriptional levels of *has1* mRNA, *has2* mRNA, and *has3* mRNA, wherein A represents effects of different concentrations of 2'-FL on the transcriptional level of *has1* mRNA, B represents effects of different concentrations of 2'-FL on the transcriptional level of *has2* mRNA, and C represents effects of different concentrations of 2'-FL on the transcriptional level of *has3* mRNA. As shown in FIG. 2, all three selected intervention concentrations of 2'-FL can significantly upregulate the transcriptional levels of *has1*, *has2*, and *has3* (*p* < 0.05). An upregulation effect of 2'-FL on the transcriptional levels of *has1*, *has2*, and *has3* decreases with increasing concentration, reaching a maximum at 0.6 mg/mL.

### 1.2.3 Effects of different concentrations of 2'-FL on heparan sulfate

The *ext1* and *ext2* genes are involved in elongation of heparan sulfate (HS) chains and are responsible for integrating the HS chains with nucleotide sugars in a Golgi apparatus. Heparan sulfate (HS) and hyaluronic acid (HA) are main glycosaminoglycan components in the glycocalyx. HS synthesis supports organogenesis, growth factor signaling, and bacterial adhesion. FIG. 3 shows effects of different concentrations of 2'-FL on transcriptional levels of *ext1* mRNA and *ext2* mRNA, wherein A represents the effects of different concentrations of 2'-FL on the transcriptional level of *ext1* mRNA, and B represents the effects of different concentrations of 2'-FL on the transcriptional level of *ext2* mRNA. As shown in FIG. 3, all three selected intervention concentrations of 2'-FL can significantly upregulate the transcriptional levels of *ext1* and *ext2* (*p* < 0.05). An upregulation effect of 2'-FL decreases with increasing concentration, reaching a maximum at 0.6 mg/mL.

### Example 2

### Preventive effects of 2'-FL on glycocalyx damage caused by LPS

2.1 Experiments: Caco-2 cells were adjusted to a density of 1 × 10⁵ cells/cm² and inoculated in 24-well plates. According to groups, 2'-FL at concentrations of 0.6 mg/mL, 1.2 mg/mL, and 2.4 mg/mL was added to MEM complete medium, respectively. After incubation for 3 days, culture supernatant was removed, and fresh MEM complete medium with a final concentration of 100 ng/mL LPS was added and they are co-incubated for 24 h. Cells with added LPS but without 2'-FL were designated as a model group, and cells without added LPS or 2'-FL were designated as a blank group. Total RNA was extracted from the cells and reverse transcribed. Transcriptional levels of genes related to glycocalyx development, including glypican 1 (*gpc1*), hyaluronic acid synthases (*has1*, *has2* and *has3*), and exostosin glycosyltransferases (*ext1* and *ext2*), in the Caco-2 cells were measured by qPCR. The qPCR primer sequences were shown in Table 1 above.

LPS (lipopolysaccharide) is a molecule that exists in bacterial cell walls and can trigger immune responses and lead to inflammation. When LPS enters the body, it may activate the immune system, leading to an inflammatory response. In some cases, LPS may induce an inflammatory response, thereby causing damage to the glycocalyx (a complex of glycoproteins, saccharides, and proteins).

### 2.2 Test Results

### 2.2.1 Preventive effects of different concentrations of 2'-FL on damage to protein backbone of glycocalyx

FIG. 4 shows effects of different concentrations of 2'-FL and LPS on the transcriptional level of *gpc1* mRNA. As can be seen from FIG. 4, after intervention with LPS, the transcription level of *gpc1* mRNA in the model group is downregulated by 2-fold compared to the blank group, indicating that LPS may have caused damage to the protein backbone of glycocalyx by downregulating the transcription level of *gpc1*. After Caco-2 cells were co-cultured with different concentrations of 2'-FL for 3 days and then intervened with LPS, it was found that the transcriptional level of *gpc1* is still upregulated compared with the blank group, wherein the concentrations of 0.6 mg/mL and 1.2 mg/mL of 2'-FL significantly upregulate the transcriptional level of *gpc1*, indicating that 2'-FL can prevent the downregulation of the transcriptional level of *gpc1* caused by LPS, further protecting the protein backbone of glycocalyx.

### 2.2.2 Preventive effects of 2'-FL on damage to hyaluronic acid synthase of glycocalyx in Caco-2 cells caused by LPS.

FIG. 5 shows effects of different concentrations of 2'-FL and LPS on the transcriptional levels of *has1* mRNA, *has2* mRNA, and *has3* mRNA, wherein A represents the effects of different concentrations of 2'-FL and LPS on the transcriptional level of *has1* mRNA, B represents the effects of different concentrations of 2'-FL and LPS on the transcriptional level of *has2* mRNA, and C represents the effects of different concentrations of 2'-FL and LPS on the transcriptional level of *has3* mRNA.

As shown in FIG. 5, after LPS intervention, the transcription levels of *has1* in the model group and the prevention groups with different concentrations of 2'-FL are significantly downregulated compared with the blank group (p < 0.05), indicating that LPS may have caused damage to hyaluronic acid synthase 1 in Caco-2 cells by downregulating the transcription level of *has1*. Furthermore, the transcription level of *has1* is still significantly different from that in normal cells after prevention with 2'-FL (p < 0.05), but after prevention with 2.4 mg/mL 2'-FL, the transcription level of *has1* is significantly upregulated compared with the model group, indicating that 2.4 mg/mL 2'-FL can prevent the decrease in transcription level of *has1* caused by LPS.

After intervention with LPS, the transcription levels of *has2* and *has3* in the model group all decreased by 2-fold compared with the blank group, indicating that LPS may cause damage to hyaluronic acid synthases 2 and 3 in Caco-2 cells by downregulating the transcription levels of *has2* and *has3*. There were no significant differences in the transcriptional levels of *has2* and *has3* between the prevention group with different concentrations of 2'-FL and the blank group (p > 0.05). Furthermore, after prevention with 2.4 mg/mL 2'-FL, the transcriptional levels of *has2* and *has3* are significantly upregulated compared to the model group (p < 0.05), indicating that 2'-FL can prevent the downregulation of the transcriptional levels of *has2* and *has3* caused by LPS, thereby maintaining the synthesis of hyaluronic acid of glycocalyx.

2.2.3 Preventive effects of 2'-FL on damage to heparan sulfate of glycocalyx in Caco-2 cells induced by LPS.

FIG. 6 shows effects of different concentrations of 2'-FL and LPS on transcriptional levels of *ext1* mRNA and *ext2* mRNA, wherein A represents the effects of different concentrations of 2'-FL and LPS on the transcriptional level of *ext1* mRNA, and B represents the effects of different concentrations of 2'-FL and LPS on the transcriptional level of *ext2* mRNA.

As can be seen from FIG. 6, after intervention with LPS, the transcription levels of *ext1* and *ext2* in the model group all decreased by 2-fold compared with the blank group, indicating that LPS may cause damage to heparan sulfate of glycocalyx by downregulating the transcription levels of *ext1* and *ext2*. After Caco-2 cells were co-cultured with different concentrations of 2'-FL for 3 days, and then intervened with LPS, it was found that the transcriptional levels of *ext1* and *ext2* are still upregulated compared with the blank group. The intervention groups with three concentrations of 2'-FL can significantly upregulate the transcriptional level of *ext1* (p<0.05), and the intervention groups with 1.2 mg/mL and 2.4 mg/mL concentrations of 2'-FL can significantly upregulate the transcriptional level of *ext2* (p<0.05), indicating that 2'-FL can prevent the downregulation of transcriptional levels of *ext1* and *ext2* caused by LPS, thereby maintaining the synthesis of heparan sulfate of glycocalyx.

The above descriptions are preferred embodiments of the present invention. It should be noted that those ordinarily skilled in the art can make various improvements and modifications without departing from the principles described herein, and these improvements and modifications should also be considered within the scope of protection of the present invention.

### INDUSTRIAL APPLICABILITY

The present invention provides use of 2'-fucosyllactose. Before adherent growth of Caco-2 cells herein, adding 2'-FL for co-culture with the Caco-2 cells can simulate the state of the intestine when growth has matured. In the state, 2'-FL can significantly promote the development of the glycocalyx layer of Caco-2 cells, and can further prevent the glycocalyx layer damage caused by LPS on this basis, showing that supplementing with a specific dose of 2'-FL during an intestinal maturation stage can significantly promote development of the intestinal glycocalyx layer and promote overall healthy development of the intestine, preventing damage from external factors such as LPS.

## Claims

1. Use of 2'-fucosyllactose in preparation of a food for promoting intestinal glycocalyx development and/or preventing glycocalyx damage caused by lipopolysaccharide (LPS).

2. Use of 2'-fucosyllactose in preparation of a drug for promoting intestinal glycocalyx development and/or preventing glycocalyx damage caused by lipopolysaccharide (LPS).

3. Use of 2'-fucosyllactose in promoting intestinal glycocalyx development and/or preventing glycocalyx damage caused by lipopolysaccharide (LPS).

4. The use according to any one of claims 1 to 3, wherein a performance of the promoting intestinal glycocalyx development is at least one selected from:
protecting a protein backbone of glycocalyx, maintaining normal synthesis of hyaluronic acid of glycocalyx, and maintaining normal synthesis of heparan sulfate of glycocalyx.

5. The use according to any one of claims 1 to 4, wherein a performance of the promoting intestinal glycocalyx development is at least one selected from: upregulating a transcriptional level of glycocalyx development-related gene glypican 1, upregulating a transcriptional level of a gene encoding hyaluronic acid synthase, and upregulating a transcriptional level of a gene encoding exostosin glycosyltransferase.

6. The use according to any one of claims 1 to 5, wherein a performance of the promoting intestinal glycocalyx development is at least one selected from: upregulating a level of *gpc1* mRNA, a level of *has1* mRNA, a level of *has2* mRNA, a level of *has3* mRNA, a level of *ext1* mRNA, and a level of *ext2* mRNA.

7. The use according to any one of claims 1 to 3, wherein a performance of the preventing glycocalyx damage caused by lipopolysaccharide (LPS) is at least one selected from:
preventing damage of protein backbone of glycocalyx caused by LPS, preventing damage of hyaluronic acid synthase of glycocalyx caused by LPS, and preventing damage of heparan sulfate of glycocalyx caused by LPS.

8. The use according to any one of claims 1 to 3 or claim 7, wherein a performance of the preventing glycocalyx damage caused by lipopolysaccharide (LPS) is at least one selected from:
upregulating a decline in a transcriptional level of glycocalyx development-related gene glypican 1 caused by LPS, upregulating a decline in a transcriptional level of a gene encoding hyaluronic acid synthase caused by LPS, and upregulating a decline in a transcriptional level of a gene encoding exostosin glycosyltransferase caused by LPS.

9. The use according to any one of claims 1 to 6 or any one of claims 7 to 8, wherein a performance of the preventing glycocalyx damage caused by lipopolysaccharide (LPS) is selected from upregulating a decline in a level of at least one of following mRNAs caused by LPS:
*gpc1* mRNA, *has1* mRNA, *has2* mRNA, *has3* mRNA, *ext1* mRNA, and *ext2* mRNA.

10. The use according to any one of claims 1 to 9, wherein a use concentration of 2'-fucosyllactose is 0.6 mg/mL-2.4 mg/mL.

11. The use according to any one of claims 1 to 9, wherein a use concentration of 2'-fucosyllactose is 0.6 mg/mL-1.2 mg/mL.

12. The use according to any one of claims 1 to 9, wherein a use concentration of 2'-fucosyllactose is 1.2 mg/mL-2.4 mg/mL.

13. Use of 2'-fucosyllactose in preparation of a food for an adjunctive treatment and/or adjunctive prevention of a gastrointestinal infection, an inflammatory bowel disease, obesity, or allergy caused by glycocalyx damage.

14. The use according to claim 1 or claim 13, wherein the food is at least one selected from the group consisting of infant formula milk powder, milk powder for kids, adult milk powder, a food for a special medical purpose, a nutritional product, liquid milk, a complementary food, and a non-alcoholic beverage.

15. The use according to any one of claims 13 to 14, wherein a use concentration of 2'-fucosyllactose is 0.6 mg/mL-2.4 mg/mL.

16. Use of 2'-fucosyllactose in the adjunctive treatment and/or adjunctive prevention of a gastrointestinal infection, an inflammatory bowel disease, obesity, or allergy caused by glycocalyx damage.
